**Europäisches Patentamt**

**European Patent Office**

**Office européen des brevets**

(19)

(11) **EP 1 252 879 A1**

(12) **EUROPEAN PATENT APPLICATION**

(43) Date of publication:
**30.10.2002 Bulletin 2002/44**

(51) Int Cl.⁷: **A61K 7/48**, A61K 7/00

(21) Application number: **01201569.9**

(22) Date of filing: **27.04.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Cosmoferm B.V.**
**2600 MA Delft (NL)**

(72) Inventors:
 • **Edens, Luppo**
 **3062 JL Rotterdam (NL)**

 • **Van der Heijden, Luc**
 **2317 NW Leiden (NL)**
 • **Vis, Albert Jon**
 **3039 WL Rotterdam (NL)**

(74) Representative: **Luys, Marie-José A.H. et al**
 **Gevers & Vander Haeghen,**
 **Hollidaystraat 5**
 **1831 Diegem (BE)**

(54) **Stabilised enzyme compositions**

(57) The present invention relates to a stabilised enzyme composition comprising a polyol and an acrylamide polymer thickening agent. The concentration of the polyacrylamide in the composition varies from 0.05 to 15 wt. %.

**EP 1 252 879 A1**

Printed by Jouve, 75001 PARIS (FR)

## Description

### Field of the invention

The present invention relates to a composition suitable for topical application, comprising an amount of an enzyme and an amount of a polyol for stabilising the enzyme.

### Background of the invention

Topical application of enzymes has found numerous applications in the cosmetic as well as in the pharmaceutical field. Superoxide dismutase and catalase enzymes for example have often been quoted in fighting radicals that are generated upon exposure to UV light (US-A-4.129.644). Glycosidases and lysozyme are thought capable of enhancing the process of skin desquamation (WO93/19731) or have been used in the treatment of acne (HUT 057608). Several patent applications disclose the use of the enzyme transglutaminase (WO94/18945, JP02204407) as well as applications involving lipolytic enzymes to control greasiness of hair (DE19824072). Combining topical application of photolyase with light therapy has been reported to be capable of repairing DNA damage in UV-irradiated human skin (PNAS 97, 1790-1795 (2000)). The use of protease enzymes has been suggested to replace $\alpha$-hydroxy acids in skin peeling formulations (JP04027388) and to support actives like retinoic acid in the treatment of acne (WO/9848775). The commercial potential of the use of proteases in cosmetic applications to soften and smoothen the skin has been enhanced by recent production process developments that enable the production of hypoallergenic versions of various proteases.

Application of enzymes in consumer products implies the need to provide enzymes with a long-term stability at ambient temperatures in aqueous conditions as the plurality of consumer products is water based. One is then however confronted with the problem that it is inherent to active enzymes that they are rather labile and show limited shelf stability. To remedy this, often a stabiliser is often added to the enzyme composition. Examples of frequently used stabilisers are the so-called polyols, which are mostly added in relatively high concentrations and may be combined with other stabilisers such as metal ions like calcium or reducing agents, the latter mostly being used in relatively low concentrations.

The primary role of polyols in stabilising enzyme containing formulations is to lower the water activity of the formulation. Although other compounds can be used for this purpose, polyols are preferred as they combine various advantages such as a good compatibility with various biological systems and a good solubility in water. The desired long-term stability of the enzymes containing formulations is achieved by maintaining the water activity level rather low, such that the polyol concentration may be increased to 40% (v/v) or more. However, the presence of high polyol concentrations in compositions for topical use is in general considered unacceptable.

To allow maintaining their active structure, many enzymes in addition to the presence of a polyol, require the presence of divalent cations, usually calcium ions. Low concentrations suffice to obtain the necessary stabilisation. The stabilising effect is achieved because these cations show a strong bonding to specific binding sites on the surface of the enzyme molecules. A similar stabilisation may be obtained with so-called non-specific ion effects, but the ion concentrations required to achieve these effects are much higher. This is undesirable as a high ion concentration is usually incompatible with galenic formulation demands.

Stabilising the enzyme with a polyol entails a temporary loss of the enzyme activity. The enzyme activity may be restored upon addition of water. However, the amount of water contained in polyol-stabilised enzyme compositions is mostly insufficient to allow reactivation of the enzyme upon topical application of the formulation. A dispensing system with which simultaneously the shelf life of stabilised enzyme compositions with low water activity may be enhanced, and enzyme activity may be restored upon application to the skin, is disclosed in WO97/27841. The dispensing system disclosed in WO97/27841 enables simultaneous delivery of two components of an aqueous composition: a first composition comprising stably formulated enzyme and a second aqueous composition. Upon delivery, both aqueous compositions are mixed, either *in situ* or in the dispensing system in such a way that a final composition is obtained which contains the enzyme in an active form and is suitable for direct topical use.

When preparing enzyme compositions for personal care one is confronted with the additional problem that the compositions, besides providing a sufficiently long shelf stability, should be presented to the consumer as attractive formulations that are convenient to use. To achieve this, the enzyme formulation contained in the dispensing system needs to have a certain minimum viscosity, which may be obtained by adding a suitable thickening agent to the enzyme formulation. Unfortunately, many of the thickening agents known in the art appear to be incompatible with enzyme compositions with a low calcium ion concentration and a high polyol concentration. It has often been encountered that the thickening effect is insufficient, as a consequence of which a formulation with an unattractive appearance is obtained which is undesireable for cosmetic applications.

## Summary of the invention.

There is thus a need to provide a stabile enzyme composition, with a sufficiently high viscosity and a cosmetically attractive appearance.

## Brief description of the invention.

This is achieved with the present invention in that the composition contains an amount of a polyacrylamide thickener.

In this respect it has been found that the incorporation of a polyacrylamide thickening agent allows increasing the viscosity of a polyol stabilised enzyme composition in such a manner that the composition looks attractive to the consumer, while simultaneously maintaining the shelf stability of the enzyme and of the composition. It has been found that polyacrylamides show a surprisingly fast hydration and quick gelling.

As the polyacrylamide thickener is virtually free of carboxylic acid groups, the tendency to form a complex with calcium ions or other divalent cations, will be negligible. Divalent cations, in particular $Ca^{2+}$ are added to enzyme compositions to stabilise the enzyme. As the tendency of the polyacrylamide thickening agent of this invention to form a complex with calcium ions or other di-valent cations is negligible, this effect needs not be compensated through addition of an extra amount of $Ca^{2+}$. As a consequence, a lower level of $Ca^{2+}$ will suffice without this adversely affecting the stability of the enzyme. This is desirable in galenic compositions.

Simultaneously with improving the shelf stability, discoloration, unwanted inhomogeneity, threadiness and tackyness of the composition are minimised, thus improving the cosmetic attractiveness of the composition.

The composition of this invention preferably comprises 0.05 to 15 weight % of the acrylamide polymer. At a concentration below 0.05 wt. %, often an insufficient thickening is observed, at a concentration above 15 wt. % the viscosity gets too high. Preferred polyacrylamide concentrations range from 0.1-10 wt. %, more preferably 0.2-5 wt.% or 0.4-2.5 wt%, although 0.8-2 wt. % is a particularly preferred concentratin range.

## Detailed description of the invention.

Polyacrylamide compounds suitable for use with the present invention are compounds corresponding to the formula:

$$- (CH_2CR(CONH_2)-$$

in which R may be H, $CH_3$. Polyacrylamide compounds suitable for use with the present invention thus include acrylamide as well as methacrylamide polymers.

The acrylamide polymer to be used in the composition of the invention may be any acrylamide polymer, provided it is substantially free of compounds or groups capable of binding $Ca^{2+}$ ions, such as for example COOH groups. It is important that the capacity of the acrylamide polymer to bind or chelate $Ca^{2+}$ ions is as low as possible to ensure a sufficient stabilisation of the enzyme. In this context, a low capacity of binding $Ca^{2+}$ ions means that the capacity of the acrylamide polymer to bind $Ca^{2+}$ ions is less than 80, preferably less than 20, more preferably less than 5 milligrams of $Ca^{2+}$ ions per gram of acrylamide polymer.

It is desirable to use acrylamide polymers that are as pure as possible, of which the monomer content has been minimised. Acrylamide monomers have been found to be toxic, as a consequence it is preferred to limit their concentration to below 2-5 ppm.

The polyacrylamide thickening agent may optionally contain agents with additional thickening properties. Suitable examples include hydrocolloids, for example xanthan, alginate, pectins or modified cellulose, for example hydroxycellulose. It should however be understood that the main thickening agent of the present invention is the acrylamide polymer.

In a preferred embodiment of the invention, the composition contains an amount of a reducing agent to protect the enzyme against destructive oxidation. Examples of suitable reducing agents for use in the composition of this invention include thiol compound, for example dithiotreitol, betmercaptoethanol, an amino acid, for example methionine, cysteine. Suitable concentrations of the reducing compounds range from 0.1 to 100 millimoles/liter of the composition. To minimise sulphur odours, preferred concentrations range from 1 to 30 millimoles/liter of the composition. The preferred reducing agent is methionine, as with the protease activity amino acids are released.

In another preferred embodiment, the composition of the invention additionally contains an amount of divalent cations, preferably $Ca^{2+}$ ions to further provide the stabilisation of the enzyme. To guarantee adequate shelf stability, the divalent cations should preferably be present in concentrations higher than 0.02 millimoles/liter, more preferably higher than 1 millimole/liter of the composition, of course available to the enzyme and not bound to the acrylamide polymer.

The polyol is preferably incorporated in the composition in a high concentration, i.e. a concentration which results in a sufficiently low water activity in the enzyme composition to adequately stabilise the enzyme. It is known in the art that these concentrations may somewhat vary with the polyol used. Preferably, the polyol is used in a concentration of 20-90 wt. % with respect to the total weight of the composition, preferably in a concentration of 30-90%, more preferably in a concentration of 50-90%, in particular 60-80%. The low water activity of the composition thus obtained is advantageous for preventing bacterial outgrowth in the composition so that a fungicide like propylparabens, which is often used in cosmetic applications, is adequate for total microbial preservation of the composition.

The type of the polyol which is used as a stabilisation agent is not critical for the invention, and may be any polyol known to the person skilled in the art, capable of effectively stabilising enzymes in aqueous compositions. Particularly suitable polyols are polyols selected from the group of glycerol, sorbitol, propylene glycol, butylene glycol, maltodextrins, or a sugar such as sucrose, lactose, glucose or trehalose. For topical applications, one should consider a polyol which is acceptable for topical use, i.e. glycerol, polyethylene glycol, butylene glycol, propylene glycol, trehalose or sorbitol.

The form in which the acrylamide polymer is incorporated into the composition may vary and is not essential to the invention. The acrylamide polymer may for example be added in the form of an emulsion, an aqueous solution or even in a granular form that later on is dissolved.

In a preferred embodiment of the invention, the acrylamide polymer is emulsified in a galenically acceptable carrier, for example isoparaffin. An example of such a product is Sepigel 305 (Seppic, Paris, France). Suitable concentrations Sepigel 305 for viscosifying the polyol-stabilized enzyme composition range from 0.1-10 wt. %, 0.2 to 5.0%, preferably from 0.4 to 2.5%, more preferably from 0.8 to 2.0% (w/w).

Obviously the molecular weight of the polyacrylamide-based thickener used is also an important parameter in establishing the final viscosity of the solution. Suitable preparations contain polyacrylamide molecules with molecular weights ranging from 1 tot 40 million Daltons, preferably from 5 to 20 million Daltons.

The enzyme contained in the composition of this invention may be any enzyme used in cosmetic compositions, for example belonging to the group of hydrolase, oxidoreductase, transferase or isomerase enzymes. More preferably, the enzyme is a protease, phosphatase, phytase, glycosidase, glucanase, mutanase ($\alpha$-1,3-glucanase), dextranase, lysozyme, lipase, phospholipase, sulfatase, urease, glucose oxidase, peroxidase, lipoxygenase, superoxide dismutase, catalase, tyrosinase, transglutaminase, photolyase or protein disulfide isomerase. The enzyme contained in the composition of this invention may also be a mixture of two or more enzymes. The preferred enzyme is a protease enzyme.

The concentration in which the enzyme is present in the composition of the invention will depend on the intended application, and will mostly vary from 10 mg to 10 g of pure protein per kg formulation. When used with the two compartment dispensing system described below, the enzyme composition is mixed with a second composition upon application. This mixing involves that the enzyme concentration is diluted, for example with a factor 10, although this may vary with the intended application.

The present invention also envisages enzyme compositions in which the enzyme is formulated in particulate form. However, in this approach an additional suspending agent may be required to prevent sedimentation of the enzyme particles. An important advantage of enzymes formulated, as particulate matter is that the risk of allergenic sensitisation is greatly reduced. Allergenic sensitisation is known to exist upon inhalation of dust of proteinaceous materials. To minimise the risk of inhalation, the enzyme is preferably formulated as particles having a particle size of at least about 5-10 $\mu$m. The upper limit of the particle size of the enzyme particles generally will be determined by the fact that larger particles may produce a gritty feeling upon application to the skin. Conveniently, the upper limit of the particle size is about 100 $\mu$m. One method to obtain enzyme particles of at least about 5-10 $\mu$m is to covalently immobilize the enzyme onto or into a suitable carrier, as described in e.g. Methods in Enzymology, vol. 44 (1976).

Another example of a suitable particle form is a crystalline enzyme with or without additional cross-linking to preserve the integrity of the particle. A commercially available form of such a cross-linked enzyme crystal is a so-called ChiroCLEC (Altus Biologics Inc., Cambridge, MA, USA). An example of an enzyme crystal protected by additional encapsulation is described in Langmuir 2000, 16, 1485-1488.

The enzyme composition of this invention is especially suitable for use in multicompartment dispensing systems. In a preferred embodiment, the enzyme compositions of the invention are topically applied using the multicompartment dispensing system disclosed in for instance WO97/27841. The dispensing system to be used is not critical to the invention. Any dispensing system is contemplated which allows for the separate containment of the stabilised enzyme composition and the second composition. Separate containment is understood to include any form of separation enabling to prevent a substantial diffusion of water from one to the other composition. The multicompartment dispensing system can be either a single dosage or a multi dosage dispensing system, for instance a dispensing system as referred to in US 6,117,433.

The use of a multicompartment dispensing system advantageously enables the simultaneous delivery of a stabilised enzyme composition and a second aqueous composition. Upon delivery, the stabilised enzyme composition and the aqueous composition are mixed, either in the dispensing system or *in situ*, resulting in a final, effective composition comprising the enzyme in an effective concentration and environment. The use of a multicompartment dispensing system allows for the dilution of the stabilising polyol present in the enzyme composition upon mixing with the second composition.

The dilution factor of the composition containing the enzyme into the second composition should be adequately chosen, i.e. taking into account the polyol concentration in the enzyme composition (and possibly the aqueous composition) so that the end concentration of the polyol does not preclude enzyme activity in the final composition. The dilution factor is determined by the ratio in which the enzyme composition and the second composition are delivered by the dispensing system. Preferably, the ratio between the enzyme composition and the second composition varies from 1:1 to 1:50, more preferably from 1:2 to 1:20, most preferably the ratio is 1:5 to 1:10.

When using a dispensing system, the concentration of the enzyme in the enzyme composition should be such that an effective enzyme concentration is reached in the final composition obtained upon mixing.

The enzyme compositions of the invention are suitable for topical use, especially for use in cosmetics.

### Example 1

**Cosmetic properties of glycerol solutions thickened with various compounds**

In first instance tests were carried out in which various cosmetic grade thickeners were dissolved in a mixture consisting of 30% (w/w) water and 70% (w/w) glycerol only. Aim was to obtain a solution with a final viscosity not exceeding 5000 cP (shear rate 12.5 1/sec; Brookfield, spindle CS 4-29, 25 °C, 50rpm). Depending on the nature of the thickener and the recommendation of the supplier, individual thickeners were first dissolved in either pure water or pure glycerol after which the other compound was added to obtain the final mixture. The pH of the mixture was adjusted to 5.5. Once prepared, the mixtures were stored for 2 weeks at ambient temperature whereupon the appearance and the cosmetic properties of the obtained gel were evaluated. Table 1 lists the various thickeners in their final concentration plus the cosmetic properties observed for the gels obtained.

Table 1

| Thickener and final concentration | Cosmetic properties of gel obtained |
|---|---|
| Carbopol Ultrez 10; 0.5% | Good |
| Carbopol ETD 2020; 0.5% | Good |
| Xanthan Gum; 0.5% | slime-like threads |
| Chitosan; 1% | malodorous |
| Hydroxyethylcellulose; 1% | slime-like threads, tends to form foam |
| Cetyl hydroxyethylcellulose; 1% | non-thickening, yellowish |
| Hydroxypropylmethylcellulose; 1% | non-homogeneous |
| Hectorite; 1% | non-thickening, brown |
| Mg-Al-silicate; 1% | non-thickening |
| Na-Mg-silicate; 3% in combination with lactic acid to obtain pH 5.5 | good |
| Polyvinylpyrrolidone (PVP) | non-thickening |
| MVE/MA copolymer crosslinked with 1,9-decadiene | non-thickening |
| Polyacrylamide emulsion; 1.5% | good |

As follows from the data presented in Table 1, only the two carbomer products (i.e.Carbopol Ultrez and Carbopol ETD 2020), the Na-Mg-silicate (i.e. Laponit XLG) in combination with lactic acid and the polyacrylamide emulsion (i.e. Sepigel 305) are capable of forming cosmetically acceptable gels with 70% glycerol in water.

### Example 2

**Enzyme stability in cosmetically acceptable gels**

According to the results obtained in Example 1 only a few thickeners are capable of forming cosmetically acceptable gels with water containing a high percentage of glycerol. In this Example we show the shelf stability of a protease in cosmetic gels in which different thickeners have been used to reach a viscosity of approx 3000 cP (shear rate 12.5/sec; Brookfield, spindle CS 4-29, 50 rpm, 25 °C).

The protease used is subtilisin (E.C. 3.4.21.62) purified from Maxatase R powder 2,16 BYU/kg as obtained from Genencor International, Brughes, Belgium. Subtilisin was purified according to the following protocol. Crude Maxatase powder was dissolved in 30 mmol/l sodium acetate pH 5.3 and the clear solution was applied to a strong cationic gel type resin (Sp Sepharose Fast Flow; Pharmacia). Upon elution of the column with 100mmol/l sodium citrate pH 5.8, the peak representing the almost pure subtilisin was collected, pooled and lyophilized.

The lyophilized powder was dissolved in a small quantity of water and equally divided over five different formulations all containing 70% (w/w) glycerol, 0.02%(w/w) CaCl2.2aq, 0.1%(w/w) methionine and 0.1% (w/w) propylparabens. To Formula 1 no thickener was added whereas Formulae 2 to 5 were thickened to approx 5000 cP using Na-Mg-silicate (Laponit XLG, Laponite US) or carbomer (Carbopol ETD 2020 from BF Goodrich) or polyacrylamide (Sepigel 305 from Seppic) respectively. In all formulae the pH was adjusted to a value between 5.0 and 5.5.

Subsequently each one of the five formulae was split into 4 portions.

Three portions of the various formulae were carefully closed and stored at either -20°C, room temperature or in an incubator at 40°C.

The fourth portion was used immediately to determine the original proteolytic activity in each formula. This proteolytic activity was measured using a standard technique of hydrolysis of a synthetic chromogenic pepticle substrate suitable for measuring subtilisin activities such as N-succinyl-Ala-Ala-Pro-Phe-pNA (from Sigma). Enzymatic hydrolysis was followed at 25 °C, in PBS buffer pH 7.2 and with a substrate concentration of 1millimol/liter by the optical extinction at 410 nanometer. The initial rates of hydrolysis were used to quantify the proteolytic activities present in the various formulae. Then the other three portions of the various formulae were carefully closed and stored at either - 20 °C, room temperature or in an incubator at 40 °C.

After 6 weeks of incubation the remaining proteolytic activity in all formulae and in all portions (-20 °C, room temperature and 40 °C) was measured using exactly the same activity test. Only in the formula without thickener added and in the formula thickened with polyacrylamide (i.e. Sepigel 305) a less than 20% deterioration of the original proteoytic activity could be established. Remarkable was that the polyacrylamide thickened formulae exhibited minor differences only in the proteolytic activities as established in the samples kept at the -20 °C, room temperature and 40 °C regimes, implying excellent enzyme stabilities under all conditions tested.

In the other formulae thickened with either the Na-Mg-silicate or the carbomer product the proteolytic activity was found to be seriously deteriorated after the 6 weeks storage period, i.e. to levels below 50% of the original activity values. Quite surprising was that in these samples all three temperature regimes showed such inadequate stabilities.

### Claims

1.  A composition comprising an amount of at least one enzyme, an amount of a polyol for stabilising the enzyme and an amount of a thickening agent, **characterised in that** as the thickening agent use is made of an acrylamide polymer.

2.  A composition as claimed in claim 1, **characterised in that** as the acrylamide polymer use is made of a - (CH$_2$CR (CONH$_2$) - polymer, in which R is either hydrogen or a methyl group.

3.  A composition as claimed in claim 1 or 2, **characterised in that** the concentration of the polyacrylamide in the composition varies from 0.05 to 15 wt. % with respect to the total weight of the composition, preferably 0.1-10 wt. %, more preferably 0.2-5 wt.%, in particular 0.8-2 wt. %.

4.  A composition as claimed in any one of claims 1-3, **characterised in that** the acrylamide polymer comprises less than 5 ppm of acrylamide monomer.

5.  A composition as claimed in any one of claims 1-4, **characterised in that** the composition comprises an amount of an amino acid.

6.  A composition as claimed in claim 5, **characterised in that** the amino acid is selected from the group of methione and cysteine.

7.  A composition as claimed in claim 5 or 6, **characterised in that** the amino acid is present in an amount of 0.1-100 millimoles/l of the composition.

8.  A composition as claimed in any one of claims 1-7, **characterised in that** the composition comprises at least 0.02, preferably at least 1 millimole/liter of the composition of divalent cations, preferably $Ca^{2+}$ ions.

9.  A composition as claimed in any one of claims 1-8, **characterised in that** the polyol is present in a concentration of 20-90 wt% with respect to the total weight of the composition.

10. A multi-compartment dispensing system comprising a first compartment with the stabilised enzyme composition of any one of claims 1-9, and a second compartment with an aqueous composition for activating the enzyme.

# EP 1 252 879 A1

## EUROPÄISCHER RECHERCHENBERICHT

Nummer der Anmeldung

EP 01 81 0689

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | GB 410 301 A (A OLIER SA DES ETS) 17. Mai 1934 (1934-05-17) * Seite 1, Zeile 32 – Zeile 93 * | 1 | A61K35/78 B01D11/02 |
| X | US 5 800 817 A (VERGE JR ARTHUR J ET AL) 1. September 1998 (1998-09-01) * Spalte 3, Zeile 40 – Spalte 4, Zeile 8 * | 1 | |
| A | DATABASE BIOSIS 'Online! BIOSCIENCES INFORMATION SERVICE, PHILADELPHIA, PA, US; 1985 RONDINA R V D ET AL: "STANDARD METHOD FOR THE SYSTEMATIC PREPARATION AND FRACTIONATION OF PLANT EXTRACTS TO TRACE THEIR PHARMACOLOGICAL ACTIVITY AND FOR CHEMICAL STUDY" Database accession no. PREV198681046341 XP002212492 * Zusammenfassung * & ACTA FARMACEUTICA BONAERENSE, Bd. 4, Nr. 1, 1985, Seiten 3-14, ISSN: 0326-2383 | | |

| RECHERCHIERTE SACHGEBIETE (Int.Cl.7) |
|---|
| A61K |

Der vorliegende Recherchenbericht wurde für alle Patentansprüche erstellt

| Recherchenort | Abschlußdatum der Recherche | Prüfer |
|---|---|---|
| DEN HAAG | 5. September 2002 | Rempp, G |

## ANHANG ZUM EUROPÄISCHEN RECHERCHENBERICHT
## ÜBER DIE EUROPÄISCHE PATENTANMELDUNG NR.

EP 01 81 0689

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-09-2002

| Im Recherchenbericht angeführtes Patentdokument | Datum der Veröffentlichung | Mitglied(er) der Patentfamilie | Datum der Veröffentlichung |
|---|---|---|---|
| GB 410301 A | 17-05-1934 | KEINE | |
| US 5800817 A | 01-09-1998 | KEINE | |

EPO FORM P0461

Für nähere Einzelheiten zu diesem Anhang : siehe Amtsblatt des Europäischen Patentamts, Nr.12/82